# EUROPEAN PATENT APPLICATION

(11) **EP 3 659 665 A1**
(43) Date of publication of application: **03.06.2020**
(21) Application number: 17918993.1
(22) Date of filing: 26.07.2017
(51) Int. Cl.: A61M 25/10, A61M 25/00

(54) **BALLOON CATHETER**

(71) Applicant: Asahi Intecc Co., Ltd., Seto-shi, Aichi 489-0071 (JP)
(72) Inventor: MIKI Akihiro, Aichi 489-0071 (JP)
(74) Representative: Winter, Brandl, Fürniss, Hübner, Röss, Kaiser, Polte - Partnerschaft mbB
(86) International application number: PCT/JP2017/027038
(87) International publication number: WO 2019/021387

(57) **Abstract**

The present invention aims at providing a balloon catheter capable of preventing a guide wire from being caught by the joint part between the outer shaft and the inner shaft.

Solution

In the balloon catheter 1, the inner shaft 30 includes a first shaft part 60 that is made of first resin, a second shaft part 70 that is made of second resin harder than the first resin and provided on the proximal end side of the first shaft part, and a large diameter portion 80 that is provided between the first shaft part 60 and the second shaft part 70 so that the proximal end portion of the first shaft part 60 and the distal end portion of the second shaft part 70 overlap and has the outer diameter D3 larger than the outer diameter D1 of the first shaft part 60 and the outer diameter D2 of the second shaft part 70, and the large diameter portion 80 includes the joint part 90 jointing the outer shaft and the inner shaft to each other.

## Description

### TECHNICAL FIELD

The present invention relates to a balloon catheter for dilating a constricted part formed in a blood vessel or a digestive organ.

### BACKGROUND ART

Conventionally, a balloon catheter is known as a therapeutic catheter that is inserted into a constricted part formed in a blood vessel or a digestive organ to expand the constricted part. The balloon catheter mainly includes a balloon as an expansion body, an outer shaft jointed to the proximal end of the balloon, and an inner shaft disposed inside the balloon and the outer shaft. The inner shaft is used for inserting a guide wire, and an expansion lumen provided between the outer shaft and the inner shaft is used for flowing liquid (such as a contrast medium or physiological saline) for expanding the balloon.

Among such balloon catheters, some kind of balloon catheter is known, in which the outer shaft and the inner shaft are jointed to each other at the proximal end side of the balloon in order to reduce the axial deviation between the outer shaft and the inner shaft (see the following Patent Literature 1, for example).

However, in the balloon catheter of Patent Literature 1, the inner shaft is greatly curved toward the outer shaft side at the joint part between the outer shaft and the inner shaft. Therefore, when the guide wire is inserted into the inner shaft, the guide wire may be caught at the curved joint part.

### CITATION LIST

### Patent Literature

Patent Literature 1: Japanese Patent Application Laid-open No. 2013-42841

### SUMMARY OF INVENTION

### TECHNICAL PROBLEM

In view of the above-described aspect, the present invention aims at providing a balloon catheter capable of preventing a guide wire from being caught at the joint part between the outer shaft and the inner shaft.

### SOLUTION TO PROBLEM

A balloon catheter according to one aspect for solving the above-described problem includes a balloon, an outer shaft that is jointed to a proximal end of the balloon, and an inner shaft that is disposed inside the outer shaft and jointed to a distal end of the balloon, in which the inner shaft includes a first shaft part that is made of first resin, a second shaft part that is made of second resin harder than the first resin and provided on a proximal end side of the first shaft part, and a large diameter portion that is provided between the first shaft part and the second shaft part so that a proximal end portion of the first shaft part and a distal end portion of the second shaft part overlap and the large diameter portion has an outer diameter larger than an outer diameter of the first shaft part and an outer diameter of the second shaft part, and the large diameter portion includes a joint part jointing the outer shaft and the inner shaft to each other.

### ADVANTAGEOUS EFFECTS OF INVENTION

In one aspect of the above-described balloon catheter, it is possible to reduce the risk that the inner shaft is greatly curved toward the outer shaft at the joint part between the outer shaft and the inner shaft and, as a result, it is possible to reduce the possibility that the guide wire is caught at the joint part.

### BRIEF DESCRIPTION OF DRAWINGS

FIG. 1 is an overall view of a balloon catheter according to a first embodiment.
FIG. 2 is an enlarged view of a portion A in FIG. 1.
FIG. 3A is a view illustrating a B-B cross section of FIG. 2. FIG. 3B is a view illustrating a C-C cross section of FIG. 2. FIG. 3C is a view illustrating a D-D cross section of FIG. 2.
FIG. 4 is a view illustrating a part of a balloon catheter according to a second embodiment.
FIG. 5A is a view illustrating an E-E cross section of FIG. 4. FIG. 5B is a view illustrating an F-F cross section of FIG. 4. FIG. 5C is a view illustrating a G-G cross section of FIG. 4.
FIG. 6 is an overall view of a balloon catheter according to a third embodiment.
FIG. 7 is an enlarged view of a portion H in FIG. 6.
FIG. 8 is a view illustrating an I-I cross section of FIG. 7. FIG. 8B is a view illustrating a J-J cross section of FIG. 7. FIG. 8C is a view illustrating a K-K cross section of FIG. 7.
FIG. 9 is a view illustrating a part of a balloon catheter according to a fourth embodiment.
FIG. 10 is an overall view of a balloon catheter according to a fifth embodiment.
FIG. 11 is an enlarged view of a portion L in FIG. 10.

### DESCRIPTION OF ENBODIMENTS

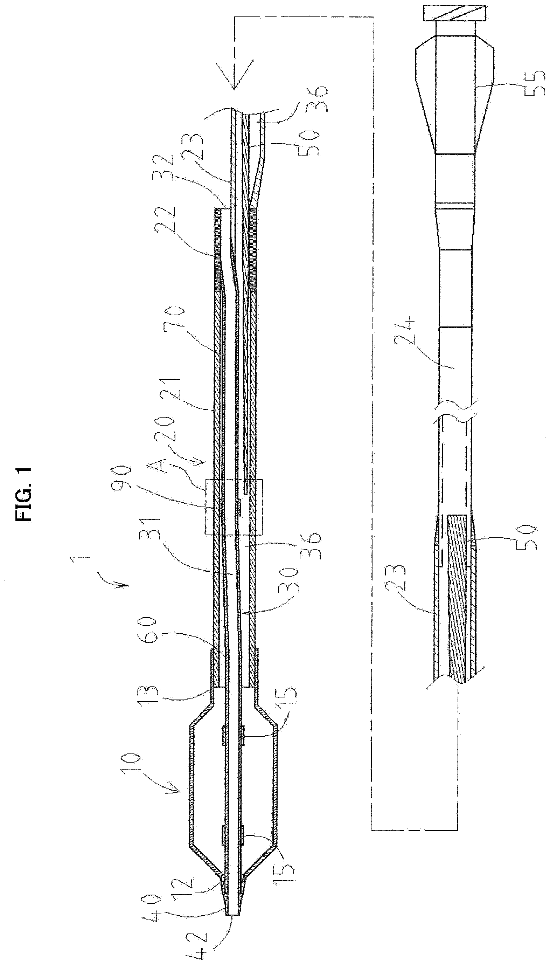

The following will describe an example using a balloon catheter 1 of the first embodiment with reference to FIGs. 1 to 3C. In FIGs. 1A and 1B, the left side in the drawings is the distal end side (far side) inserted into a body, and the right side is the proximal end side (near side) operated by a technician such as a doctor.

The balloon catheter 1 is used for expanding and treating a constricted part formed in a blood vessel or a digestive organ, for example. As illustrated in FIG. 1, the balloon catheter 1 mainly includes a balloon 10, an outer shaft 20, an inner shaft 30, a distal tip 40, a core wire 50, and a connector 55.

The balloon 10 expanding a constricted part is made of a resin member formed with materials such as a polyether block amide copolymer (nylon-based elastomer resin) and polyurethane resin, and includes a distal end attachment portion 12 on the distal end side and a proximal end attachment portion 13 on the proximal end side. The distal end attachment portion 12 is jointed to the distal end of the inner shaft 30 through the distal tip 40, and the proximal end attachment portion 13 is jointed to the distal end of the outer shaft 20. In FIG. 1, the distal tip 40 is provided between the distal end attachment portion 12 and the distal end of the inner shaft 30. However, the embodiment is not limited thereto, and the distal end attachment portion 12 may be jointed directly to the distal end of the inner shaft 30. In addition, the proximal end attachment portion 13 is jointed to the outer periphery of the distal end of the outer shaft 30. However, the embodiment is not limited thereto, and the proximal end attachment portion 13 may be jointed to the inner periphery of the distal end of the outer shaft 20. The balloon 10 may be formed of a single layer or a multilayer.

The outer shaft 20 is a tubular member that forms an expansion lumen 36 for supplying liquid such as a contrast medium or physiological saline in order to expand the balloon 10. The outer shaft 20 includes a distal end outer shaft portion 21, a guide wire port portion 22, an intermediate outer shaft portion 23, and a proximal end outer shaft portion 24 in this order from the distal end side. The distal outer shaft portion 21 and the intermediate outer shaft portion 23 are tubes made of resin such as polyamide, polyamide elastomer, polyolefin, polyester, or polyester elastomer. The guide wire port portion 22 is a portion where the proximal end of the distal end outer shaft portion 21, the distal end of the intermediate outer shaft portion 23, and the proximal end of the inner shaft 30 are jointed.

The inner shaft 30 is inserted into the distal outer shaft portion 21, and the above-described expansion lumen 36 is formed between the distal end outer shaft portion 21 and the inner shaft 30.

The proximal end outer shaft portion 24 is a metallic tubular member referred to as a so-called hypotube. The distal end of the proximal end outer shaft portion 24 is inserted and jointed to the proximal end of the intermediate outer shaft portion 23. The connector 55 is attached to the proximal end of the proximal end outer shaft portion 24. When liquid such as a contrast medium or physiological saline for expanding the balloon 10 is supplied from an indeflator (not illustrated) attachable to the connector 55, the liquid passes through the expansion lumen 36 and expands the balloon 10. The material of the proximal end outer shaft portion 24 is not particularly limited, and stainless steel (SUS304) or superelastic alloys such as Ni-Ti alloy may be used.

The inner shaft 30 forms a guide wire lumen 31 for inserting a guide wire to the inside. Further, the proximal end of the inner shaft 30 is jointed to the guide wire port portion 22 of the outer shaft 20 to form a proximal end side guide wire port 32.

The distal tip 40 is jointed to the distal end of the inner shaft 30. The distal tip 40 is made of soft resin. The material is not particularly limited, and polyurethane, polyurethane elastomer, or the like may be used. The distal tip 40 has a distal end side guide wire port 42 at the distal end.

A marker 15 having radiopacity is attached to the inner shaft 30 in the balloon 10 so that the position of the balloon 10 can be grasped under irradiation. The number and position of the marker 15 may be appropriately changed depending on the length of the balloon 10.

A core wire 50 is attached to the inner peripheral surface of the distal end of the proximal end outer shaft portion 24. The core wire 50 is a tapered metal wire rod having a circular cross section and having a diameter reduced toward the distal end. The material of the core wire 50 is not particularly limited, and stainless steel (SUS304) or superelastic alloys such as Ni-Ti alloy may be used. The core wire 50 passes the intermediate outer shaft portion 23 and the guide wire port portion 22, and extends to the distal end outer shaft portion 21.

The inner shaft 30 includes a first shaft part 60 formed of flexible first resin, and a second shaft part 70 formed of a second resin harder than the first resin on the proximal end side of the first shaft part 60. In the embodiment, a polyether block amide copolymer (nylon elastomer resin) is used for the first shaft part 60, while polyamide resin is used for the second shaft part 70. The embodiment is not limited thereto, and may be such that the first shaft part 60 and the second shaft part 70 are formed of the same resin, and the Shore hardness of the first shaft part 60 on the distal end side can be lower than the Shore hardness of the second shaft part 70on the proximal end side.

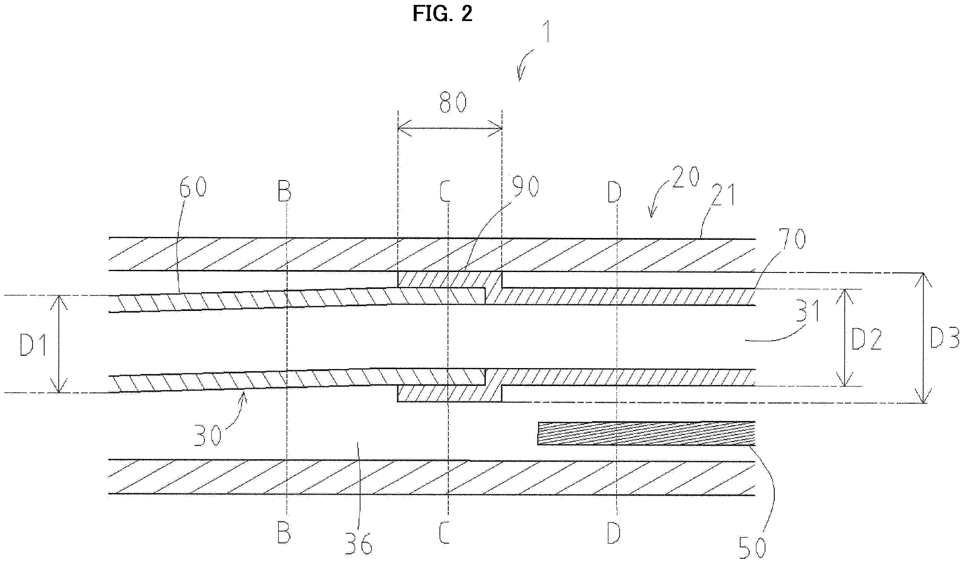

FIG. 2 is an enlarged view of a portion A in FIG. 1. As illustrated in FIG. 2, the distal end portion of the second shaft part 70 covers the proximal end portion of the first shaft part 60. Therefore, there is provided a large diameter portion 80 that is positioned between the first shaft part 60 and the second shaft part 70 so that the proximal end portion of the first shaft part 60 and the distal end portion of the second shaft part 70 overlap and the large diameter portion 80 has an outer diameter D3 larger than the outer diameter D1 of the first shaft part 60 and the outer diameter D2 of the second shaft part 70.

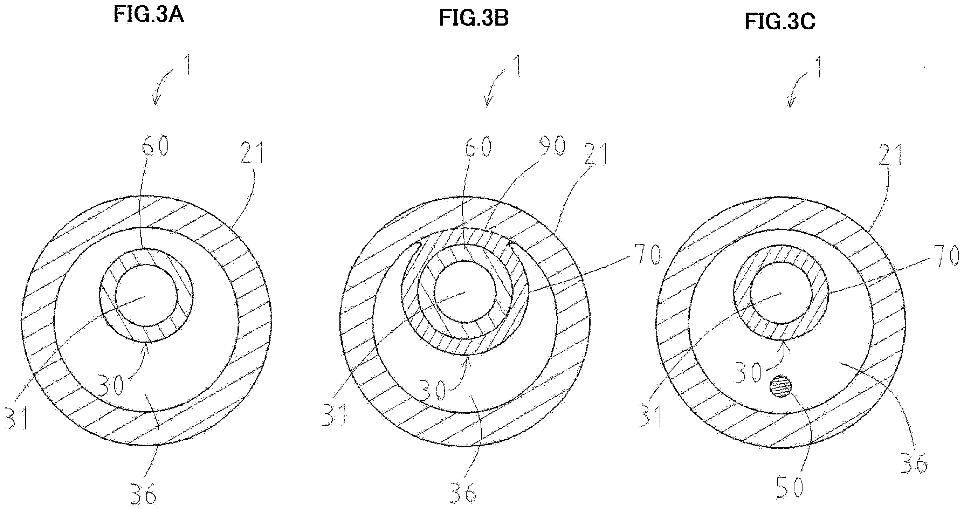

FIG. 3A is a diagram illustrating a B-B cross section of FIG. 2, FIG. 3B is a diagram illustrating a C-C cross section of FIG. 2, and FIG. 3C is a diagram illustrating a D-D cross section of FIG. 2. As illustrated in FIGs. 2 and 3B, the large diameter portion 80 includes a joint part 90 where the outer shaft 20 (distal end outer shaft portion 21) and the inner shaft 30 are jointed to each other. In other words, at the joint part 90 of the large diameter portion 80, the inner peripheral surface of the outer shaft 20 is joined to the outer peripheral surface of the distal end portion of the second shaft part 70. Meanwhile, as illustrated in FIGs. 2, 3A, and 3C, the outer shaft 20 (the distal end outer shaft portion 21) and the inner shaft 30 are not jointed to each other except at the large diameter portion 80.

Thus, in the balloon catheter 1, the outer shaft 20 and the inner shaft 30 are jointed to each other at the large diameter portion 80 having the large outer diameter D3. This reduces the possibility that the inner shaft 30 is curved greatly to the outer shaft 20 side (paper surface upper side in FIGs. 2 and 3B), which consequently reduces the possibility that the guide wire inserted into the guide wire lumen 31 is caught at the joint part 90.

Further, with the large diameter portion 80 including the joint part 90 jointing the outer shaft 20 and the inner shaft 30, it is possible to reduce the possibility that the second shaft portion 70 is pulled out from the first shaft part 60 when the balloon catheter 1 is pulled to the proximal end side. Further, in the large diameter portion 80, the proximal end portion of the first shaft part 60 and the distal end portion of the second shaft part 70 are overlapped, and thereby the thickness of the large diameter portion 80 is increased (see FIGs. 2 and 3B). Therefore, even when the joint part 90 is provided, it is easy to maintain the circularity of the guide wire lumen 31 in the large diameter portion 80, which consequently reduces the possibility that the guide wire is caught at the joint part 90.

In the balloon catheter 1, the second shaft part 70 on the proximal end side is formed of hard resin, and the first shaft part 60 on the distal end side is formed of flexible resin. Thus, when the operator pushes the balloon catheter 1 into the distal end side, the pushing force of the operator can be easily transmitted to the distal end side, thereby allowing the balloon 10 to be easily inserted into a constricted part.

In the embodiment, the joint part 90 is formed by irradiation with laser light from the outside of the outer shaft 20 in a state where the large diameter portion 80 is drawn toward the outer shaft 20 side (paper surface upper side in FIG. 2 and FIG. 3B). The method for forming the joint part 90 is not particularly limited. For example, the joint part 90 may be formed by jointing the outer shaft 20 and the inner shaft 30 (at large diameter portion 80) using an adhesive.

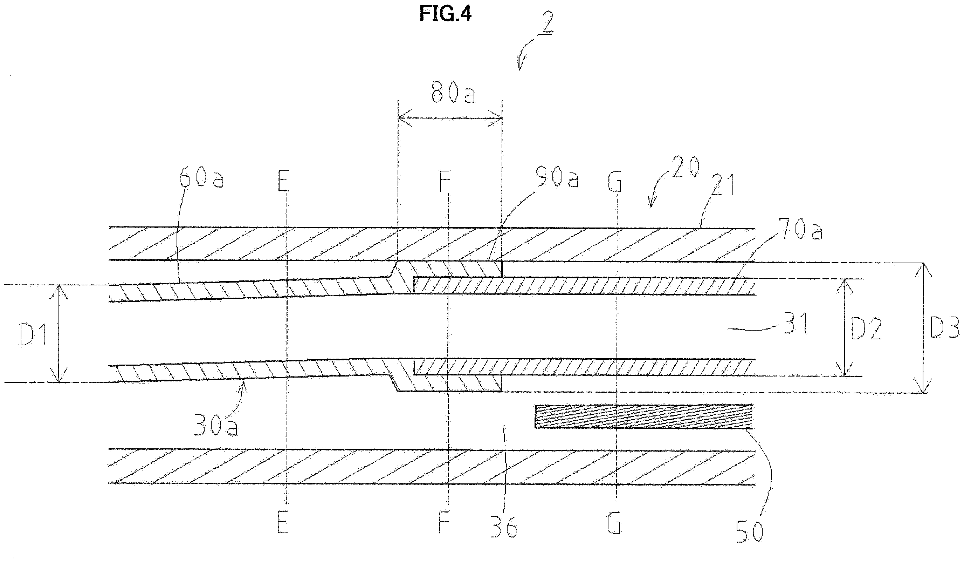

The following will describe a balloon catheter 2 of the second embodiment with reference to FIG. 4. Here, the same members as those of the balloon catheter 1 according to the first embodiment will be represented with the same reference numerals, and the detailed description thereof will be omitted. Only the differences from the balloon catheter 1 illustrated in FIG. 2 will be described. As illustrated in FIG. 4, an inner shaft 30a includes a first shaft part 60a formed of flexible first resin and a second shaft portion 70a formed of second resin harder than the first resin and provided on the proximal end side of the first shaft part 60a. The proximal end portion of the first shaft part 60a covers the distal end portion of the second shaft part 70a. Therefore, there is provided a large diameter portion 80a that is formed between the first shaft part 60a and the second shaft part 70a so that the proximal end portion of the first shaft part 60a and the distal end portion of the second shaft part 70a overlap, and thereby the large diameter portion 80a has the outer diameter D3 larger than the outer diameter D1 of the first shaft part 60a and the outer diameter D2 of the second shaft part 70a.

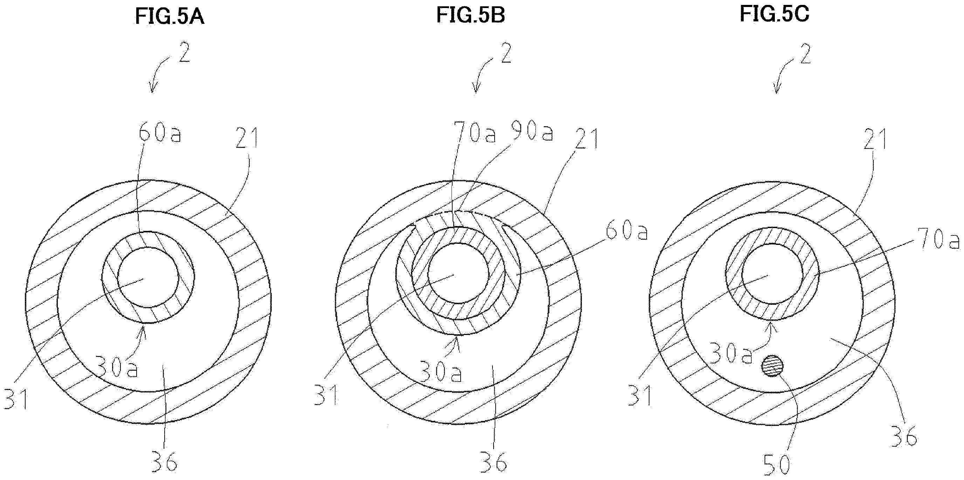

FIG. 5A is a diagram illustrating an E-E cross section of FIG. 4, FIG. 5B is a diagram illustrating an F-F cross section of FIG. 4, and FIG. 5C is a diagram illustrating a G-G cross section of FIG. 4. As illustrated in FIGs. 4 and 5B, the large diameter portion 80a includes a joint part 90a where the outer shaft 20 (distal end outer shaft portion 21) and the inner shaft 30a are jointed to each other. In other words, at the joint part 90a of the large diameter portion 80a, the inner peripheral surface of the outer shaft 20 is jointed to the outer peripheral surface of the proximal end portion of the first shaft part 60a. Meanwhile, as illustrated in FIGs. 4, 5A, and 5C, the outer shaft 20 (the distal end outer shaft portion 21) and the inner shaft 30a are not jointed to each other except at the large diameter portion 80a.

Also with this configuration, it is possible to exert the same actions and effects as the balloon catheter 1 according to the first embodiment. In addition, in the balloon catheter 2, with the hard second shaft part 70a existing on the inner side of the flexible first shaft 60a at the joint part 90a between the outer shaft 20 and the inner shaft 30a, the circularity of the guide wire lumen 31 at the joint part 90a is further secured (in other words, the crushing of the inner shaft 30a at the joint part 90a can be further reduced), which consequently reduces the possibility that the guide wire is caught at the joint part 90a.

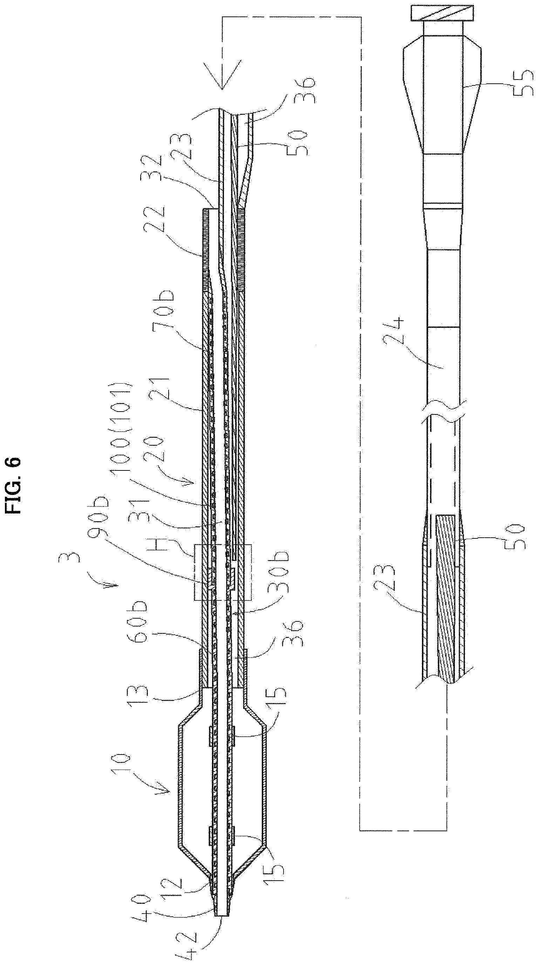

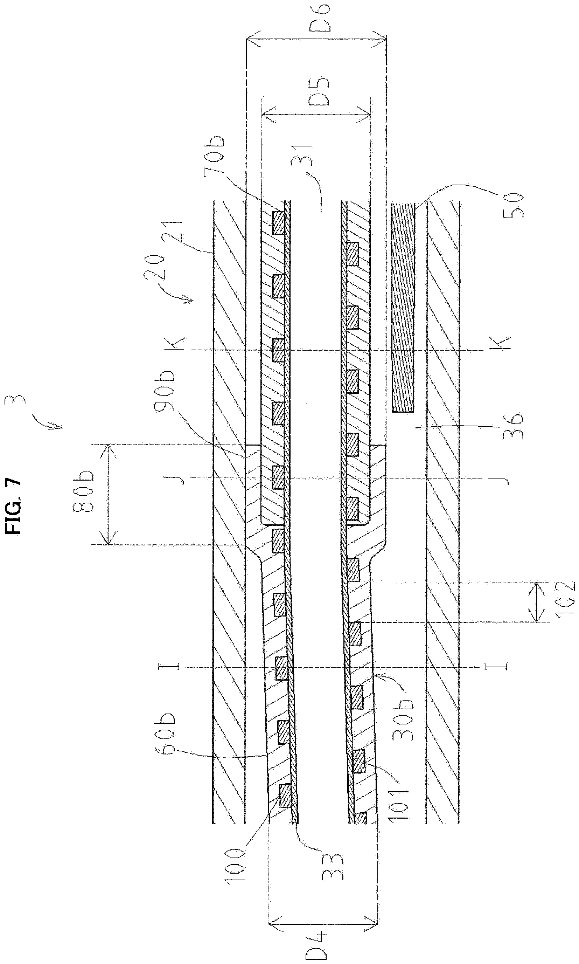

The following will describe a balloon catheter 3 of the third embodiment with reference to FIGs. 6 to 8. Here, the same members as those of the balloon catheter 1 according to the first embodiment and the balloon catheter 2 according to the second embodiment will be represented by the same reference numerals, and the detailed description thereof will be omitted. Only the differences from the balloon catheter 1 illustrated in FIG. 1 and the balloon catheter 2 illustrated in FIG. 4 will be described. As illustrated in FIGs. 6 and 7, an inner shaft 30b includes a reinforcing layer 100 that extends from a first shaft part 60b through a large diameter portion 80b to a second shaft part 70b and is wound by strands 101 having a gap 102 between adjacent strands 101. The inner shaft 30b also includes an inner layer 33 that extends from the first shaft part 60b to the large diameter portion 80b and then to the second shaft part 70b on the inner peripheral side of the reinforcing layer 100.

The material of the strand 101 forming the reinforcement layer 100 is not particularly limited, and stainless steel (SUS304), superelastic alloys such as Ni-Ti alloy, or PEEK resin may be used. In the embodiment, polytetrafluoroethylene (PTFE) is used as the inner layer 33. However, the material used for the inner layer 33 is not particularly limited.

As illustrated in FIG. 7, the inner shaft 30b includes the first shaft part 60b that covers the outer periphery of the above-described reinforcing layer 100 and is formed of flexible first resin and a second shaft part 70b that covers the outer periphery of the reinforcing layer 100 and is formed of second resin harder than the first resin on the proximal end side of the first shaft part 60b. The proximal end portion of the first shaft part 60b covers the distal end portion of the second shaft part 70b. Therefore, there is provided the large diameter portion 80b that is formed between the first shaft part 60b and the second shaft part 70b so that the proximal end portion of the first shaft part 60b and the distal end portion of the second shaft part 70b overlap, and thereby the large diameter portion 80b has an outer diameter D6 larger than an outer diameter D4 of the first shaft part 60b and an outer diameter D5 of the second shaft part 70b.

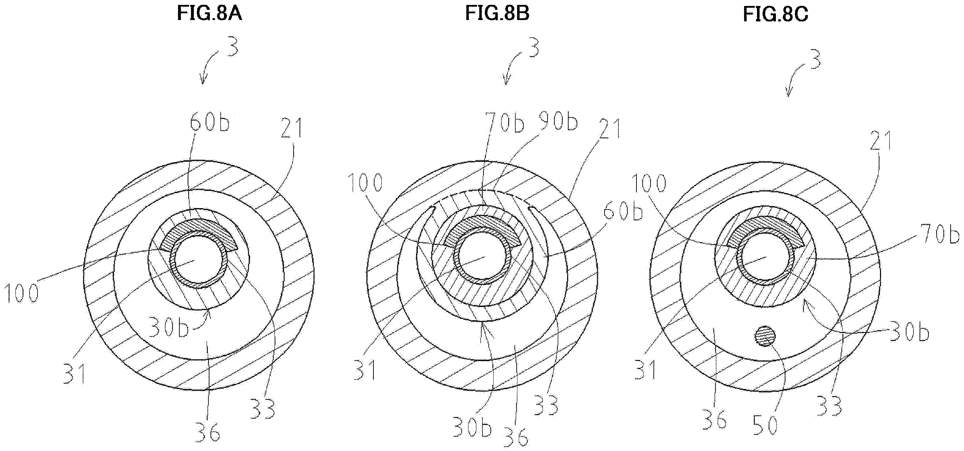

FIG. 8A is a diagram illustrating an I-I cross section in FIG. 7, FIG. 8B is a diagram illustrating a J-J cross section in FIG. 7, and FIG. 8C is a diagram illustrating a K-K cross section of FIG. 7. As illustrated in FIGs. 7 and 8B, the large diameter portion 80b includes a joint part 90b where the outer shaft 20 (distal end outer shaft portion 21) and the inner shaft 30b are jointed to each other. In other words, in the joint part 90b of the large diameter portion 80b, the inner peripheral surface of the outer shaft 20 is jointed to the outer peripheral surface of the proximal end portion of the first shaft part 60b. Meanwhile, as illustrated in FIGs. 7, 8A, and 8C, the outer shaft 20 (the distal end outer shaft portion 21) and the inner shaft 30b are not jointed to each other except at the large diameter portion 80b.

Also with this configuration, it is possible to exert the same actions and effects as the balloon catheter 1 according to the first embodiment and the balloon catheter 2 according to the second embodiment. In addition, in the balloon catheter 3, with the reinforcing layer 100 existing inside the second shaft part 70b at the joint part 90b between the outer shaft 20 and the inner shaft 30b, the circularity of the guide wire lumen 31 at the joint part 90a is further secured (in other words, the crushing of the inner shaft 30a at the joint part 90b can be further reduced), which consequently reduces the possibility that the guide wire is caught at the joint part 90b.

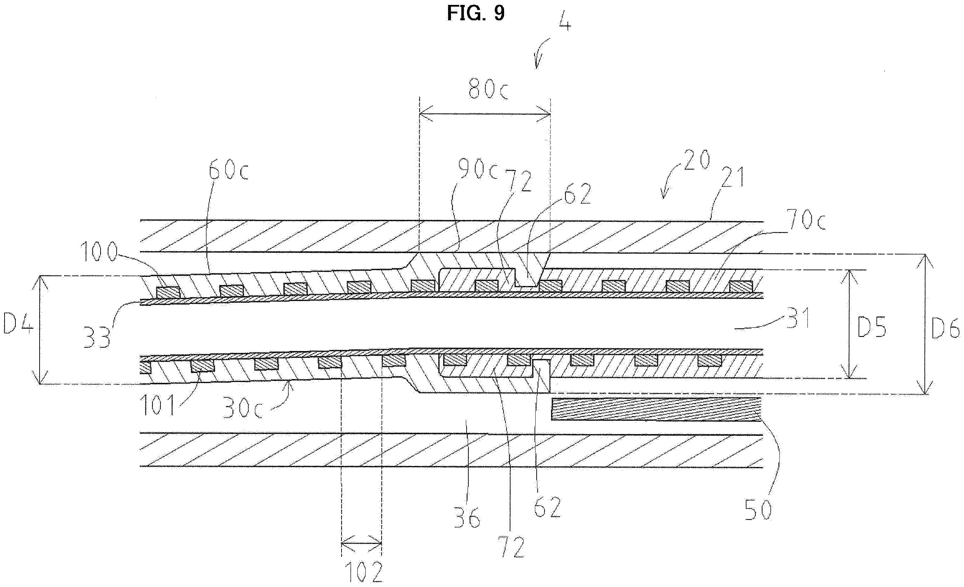

The following will describe a balloon catheter 4 of the fourth embodiment with reference to FIG. 9. Here, the same members as those of the balloon catheter 3 according to the third embodiment will be represented with the same reference numerals, and the detailed description thereof will be omitted. Only the differences from the balloon catheter 3 illustrated in FIG. 7 will be described. As illustrated in FIG. 9, an inner shaft 30c includes a first shaft part 60c that covers the outer periphery of the reinforcing layer 100 and is formed of flexible first resin and a second shaft part 70c that covers the outer periphery of the reinforcing layer 100 and is formed of second resin harder than the first resin on the proximal end side of the first shaft part 60b. A proximal end portion 62 of the first shaft part 60c covers a distal end portion 72 of the second shaft part 70c. Therefore, between the first shaft part 60c and the second shaft part 70c, there is provided a large diameter portion 80c having an outer diameter D6 larger than the outer diameter D4 of the first shaft part 60b and the outer diameter D5 of the second shaft part 70b at which the proximal end portion 62 of the first shaft part 60c and the distal end portion 72 of the second shaft part 70c overlap.

The large diameter portion 80c includes a joint part 90c where the proximal end portion 62 of the first shaft part 60c is jointed to the distal end portion 72 of the second shaft part 70c in a state where the proximal end portion 62 of the first shaft part 60c enters the gap 102 between the strands 101. Therefore, with the anchor effect between the proximal end portion 62 of the first shaft part 60c and the distal end portion 72 of the second shaft part 70c, and the anchor effect between the proximal end portion 62 of the first shaft part 60c and the strand 101 of the reinforcing layer 100, it is possible to further reduce the possibility that the second shaft part 70c is pulled out from the first shaft part 60c when the balloon catheter 4 is pulled to the proximal end side.

In the embodiment, the joint part 90c is provided by irradiation with laser light from the outside of the outer shaft 20 in a state where the large diameter portion 80c is drawn toward the outer shaft 20 side (paper surface upper side in FIG. 9). At this time, the position to be irradiated with the laser beam is adjusted (for example, only the proximal end side of the large diameter portion 80c is irradiated with the laser beam) so that the proximal end portion 62 of the first shaft part 60c is buried in the gap 102 between the strands 101. However, the method of forming the joint part 90c is not limited to this. For example, the joint part 90c may be formed by swaging the proximal end portion 62 of the first shaft part 60c from the outer periphery in a state where the proximal end portion 62 of the first shaft part 60c is covered by the front end portion 72 of the second shaft part 70c, so as to form the inner shaft 30c in which the proximal end portion 62 of the first shaft part 60c is buried in the gap 102 between the strands 101, and then jointing the inner shaft 30c and the outer shaft 20 by an adhesive.

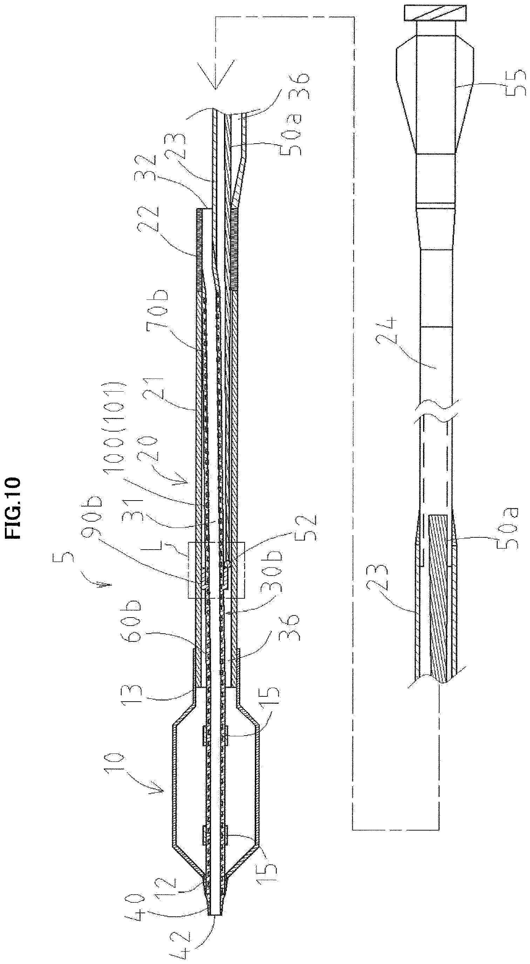

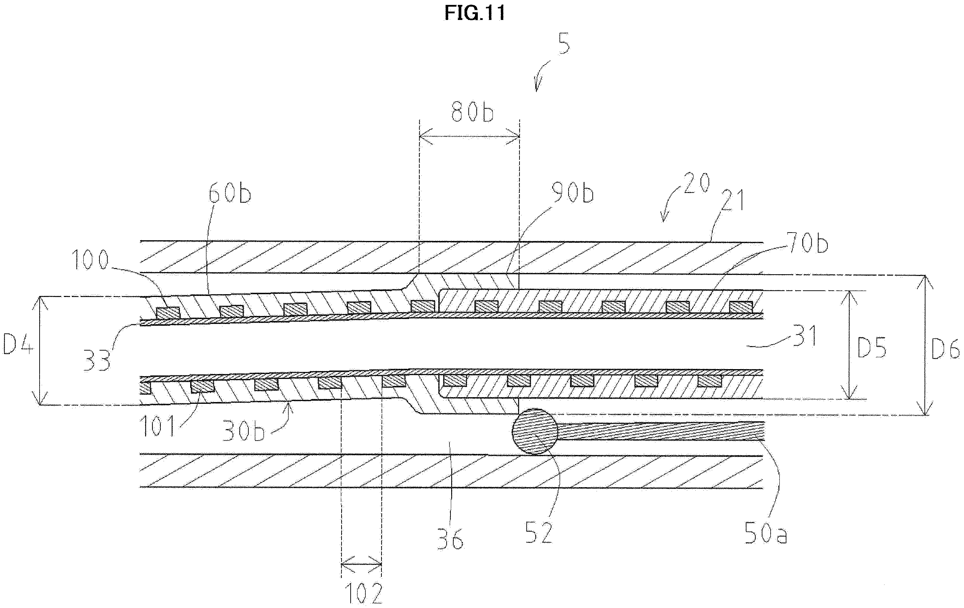

The following will describe a balloon catheter 5 of the fifth embodiment with reference to FIGs. 10 and 11. Here, the same members as those of the balloon catheter 3 according to the third embodiment will be represented with the same reference numerals, and the detailed description thereof will be omitted. Only the differences from the balloon catheter 3 illustrated in FIGs. 6 and 7 will be described. As illustrated in FIG. 10, a bulging portion 52 is provided at the distal end of a core wire 50a.

FIG. 11 is an enlarged view of a portion L in FIG. 10. As illustrated in FIG. 11, the bulging portion 52 of the core wire 50a is in contact with the large diameter portion 80b that is provided so that the proximal end portion of the first shaft part 60b and the distal end portion of the second shaft part 70b overlap and the large diameter portion 80b has the outer diameter D6 larger than the outer diameter D4 of the first shaft part 60b and the outer diameter D5 of the second shaft part 70b. Therefore, in the balloon catheter 5, when the operator pushes the connector 55 in the distal direction, the pushing force is transmitted from the bulging portion 52 of the core wire 50a to the distal end of the inner shaft 30b through the large diameter portion 80b. At this time, the outer shaft 20 and the large diameter portion 80b are jointed to each other at the joint part 90b. Thus, even when the bulging portion 52 of the core wire 50a pushes the large diameter portion 80b toward the distal end side, the first shaft part 60b is not separated from the second shaft part 70b.

The first shaft parts 60a, 60b, 60c described in the above-described second to fifth embodiments may be formed of the same resin material as that of the first shaft part 60 described in the first embodiment. Similarly, the second shaft parts 70a, 70b, 70c described in the above-described second to fifth embodiments may be formed of the same resin material as that of the second shaft part 70 described in the first embodiment.

Further, the bulging portion 52 described in the fifth embodiment may be applied to the balloon catheters 1, 2, 3, 4 described in the first to fourth embodiments.

Further, in the balloon catheters 1, 2, 3, 4, 5, the position of the joint parts 90, 90a, 90b, and 90c is not particularly limited. It may be formed, for example, slightly on the proximal end side from the proximal end attachment portion 13 of the balloon 10, or slightly on the distal end side from the guide wire port portion 22.

### REFERENCE SIGNS LIST

1, 2, 3, 4, 5 balloon catheter
10 balloon
12 distal end attachment portion
13 proximal end attachment portion
15 marker
20 outer shaft
21 distal end outer shaft portion
22 guide wire port portion
23 intermediate outer shaft portion
24 proximal end outer shaft portion
30, 30a, 30b, 30c Inner shaft
31 guide wire lumen
32 proximal end side guide wire port
33 inner layer
36 expansion lumen
40 distal tip
42 distal end side guide wire port
50, 50a core wire
52 bulging portion
55 connector
60, 60a, 60b, 60c first shaft part
62 proximal end portion
70, 70a, 70b, 70c second shaft part
80, 80a, 80b, 80c large diameter portion
90, 90a, 90b, 90c joint part
100 reinforcing layer
102 gap

## Claims

1. A balloon catheter, comprising: a balloon;
an outer shaft that is jointed to a proximal end of the balloon; and
an inner shaft that is disposed inside the outer shaft and jointed to a distal end of the balloon, wherein
the inner shaft includes
a first shaft part that is made of first resin,
a second shaft part that is made of second resin harder than the first resin and provided on a proximal end side of the first shaft part, and
a large diameter portion that is provided between the first shaft part and the second shaft part so that a proximal end portion of the first shaft part and a distal end portion of the second shaft part overlap and has an outer diameter larger than an outer diameter of the first shaft part and an outer diameter of the second shaft part, and
the large diameter portion includes a joint part jointing the outer shaft and the inner shaft to each other.

2. The balloon catheter according to claim 1, wherein the large diameter portion is formed so that the proximal end portion of the first shaft part covers the distal end portion of the second shaft part,
the outer shaft is jointed to the proximal end portion of the first shaft part at the large diameter portion.

3. The balloon catheter according to claim 1 or 2, wherein the inner shaft further includes a reinforcing layer that extends from the first shaft part through the large diameter portion to the second shaft part and is wound by strands having a gap between the adjacent strands.

4. The balloon catheter according to claim 3, wherein in the large diameter portion, the proximal end portion of the first shaft part is jointed to the distal end portion of the second shaft part in a state of entering the gap between the strands.
